# EUROPÄISCHE PATENTANMELDUNG

(11) **EP 1 604 608 A1**
(43) Veröffentlichungstag der Anmeldung: **14.12.2005**
(21) Anmeldenummer: 05104981.5
(22) Anmeldetag: 08.06.2005
(51) Int. Cl.: A61B 3/15, A61F 9/007, G02B 21/08

(54) **Retinaschutzvorrichtung für ein Operationsmikroskop**

(30) Priorität: 11.06.2004 DE 102004028470
(71) Anmelder: Leica Microsystems (Schweiz) AG, 9445 Heerbrugg (CH)
(72) Erfinder: Sander, Ulrich, 9445, Rebstein (CH)
(74) Vertreter: Reichert, Werner Franz

(57) **Zusammenfassung**

Augen-, insbesondere Retina-Schutzvorrichtung für ein Operationsmikroskop mit einem Beleuchtungsstrahlengang, der ein Leuchtfeld für eine Objektebene (14) definiert, und einem optischen Element (4) im Beleuchtungsstrahlengang, das derart ausgebildet ist, dass es Licht aus dem zentralen oder einem dezentralen Bereich des Leuchtfeldes herauslenkt. Das optische Element (4) weist vorzugsweise wenigstens eine Freiformfläche auf.

## Beschreibung

Die Erfindung betrifft eine Augen-, insbesondere Retina-Schutzvorrichtung und ein optisches Element mit einer Freiformfläche für einen Beleuchtungsstrahlengang, sowie die Verwendung eines optischen Elements mit Freiformfläche zum Schutz der Retina des Patientenauges im Verlauf von Augenoperationen. Das Patientenauge soll zeitweise vor dem Licht geschützt werden, das einem Chirurgen zur Beleuchtung des Operationsfeldes am Auge dient. Die Retina sowie die Kornea und die Linse könnten sonst durch eine zu große Bestrahlungsdosis geschädigt werden.

Üblicherweise werden in Operationsmikroskopen Filter verwendet, die schädliche spektrale Anteile des Lichts herausfiltern.

Die DE-A1-33 39 172 beschreibt eine Lichtfalle für Operationsmikroskope. Mit einer in den Beleuchtungsstrahlengang eines Operationsmikroskops ein-schwenkbaren Blende werden Zonen des Leuchtfeldes im Objektfeld abgeschattet. Bei der Abschattung der Leuchtfeldmitte im Bereich der Pupille wird verhindert, dass ein schädlicher Lichtstrahl auf die Retina des Patientenauges fällt. Es bildet sich also ein dunkler Fleck zentrisch im Leuchtfeld über der Patientenpupille ab.

Der Nachteil hierin ist aber, dass durch diese Absorption des Lichts im Zentrum des Beleuchtungsstrahlengangs durch die Blende Verlustwärme entsteht, sodass beispielsweise das Trägermaterial zerspringen könnte. Durch die lichtabsorbierende Schicht der Blende geht außerdem ein nicht unrelevanter Anteil des Lichts verloren.

Es stellt sich also die Aufgabe, eine gattungsgemäße Schutzvorrichtung bzw. ein optisches Element zu finden, durch das keine Verlustwärme entsteht. Der Erfinder löst die Aufgabe mit Hilfe der Merkmale des Anspruches 1.

Die erfindungsgemäße Augen-, insbesondere Retina-Schutzvorrichtung für ein Operationsmikroskop besteht aus einem Beleuchtungsstrahlengang, der ein Leuchtfeld für eine Objektebene definiert, und einem optischen Element im Beleuchtungsstrahlengang, welches derart ausgebildet ist, dass es Licht aus dem zentralen Bereich des Leuchtfeldes herauslenkt. In einem besonderen Falle, in welchem das Auge verrollt ist und sich die Pupille nicht im zentralen Bereich befindet, wird das Licht aus einem dieser Position ent-sprechenden dezentralen Bereich des Leuchtfeldes herausgelenkt. Durch diese Vorrichtung wird das Licht nicht im Zentrum bzw. im dezentralen Bereich in Wärme umgewandelt, sondern von dort weggeleitet. In einer besonderen Ausführung wird entweder die Intensität in dem verbleibenden Bereich des Beleuchtungsstrahlenganges verstärkt oder Licht in für die Retina unproblematische andere seitliche Bereiche des Beleuchtungsstrahlenganges geleitet, was insgesamt weniger Verlustwärme erzeugt, als die in DE-A1-33 39 172 beschriebene Absorption. Die Form der so entstehenden Abschattung im Leuchtfeld kann kongruent mit dem optischen Element sein, jedoch auch jede erdenkliche andere Form annehmen.

Ein derartiges optisches Element kann in seinem peripheren Bereich partiell einen unendlichen Radius aufweisen, sodass hier der Ablenkungseffekt entfällt.

Erfindungsgemäß ist gemäß einer Ausgestaltung die Oberfläche des optischen Elements durch eine Freiformfläche gebildet. Diese Freiformfläche kann auch nicht-rotationssymmetrische Abweichungen von der Sphäre aufweisen, vorzugsweise eine Topographie, die z.B. durch ein Polynom höherer Ordnung dargestellt wird.

So ist man in der Lage, dass mit derart verformten optischen Elementen Licht in die gewünschten Richtungen seitlich des zentralen oder dezentralen Bereiches abgelenkt werden kann. In der Mikroskopbeleuchtung wird bevorzugt anstelle der bisherigen Retina-Schutzblende ein optisches Element mit Freiformfläche verwendet, welche im zentralen oder in einem dezentralen Bereich des Beleuchtungsstrahlenganges in einer zur Objektebene konjugierten Ebene angeordnet ist.

Als weitere Ausführungsform kann ebenso eine Oberfläche der Kondensorlinsen oder einer anderen Linse als Freiformfläche ausgestaltet werden, um einen vergleichbaren Effekt zu erzielen. Damit kann auf ein zusätzliches Bauteil verzichtet werden. Das optische Element kann wahlweise aus dem Beleuchtungsstrahlengang elektrisch oder elektronisch angesteuert - gegebenenfalls ferngesteuert - motorisch eingeschwenkt werden.

Die erfindungsgemäße Augen-, insbesondere Retina-Schutzvorrichtung muss nicht ausschließlich für ein Operationsmikroskop zur Anwendung kommen, sie kann vielmehr auch in anderen optischen Geräten eingebaut sein.

Weitere Ausbildungen der Erfindung sind in den Figuren dargestellt und in den abhängigen Patentansprüchen angegeben. Die Bezugszeichenliste ist Bestandteil der Offenbarung.

Anhand der Figuren wird die Erfindung symbolisch und beispielhaft näher erläutert.

Es zeigen dabei
- Fig. 1 -: eine schematische Darstellung eines Beleuchtungsstrahlenganges in Anwendung einer Retina-Schutzblende nach dem Stand der Technik,
- Fig. 2 -: eine schematische Darstellung eines Beleuchtungsstrahlenganges in Anwendung eines erfindungsgemäßen optischen Elements, insbesondere einer speziellen Retina-Schutzlinse mit Freiformfläche,
- Fig. 3 -: eine schematische Darstellung eines Beleuchtungsstrahlenganges in Anwendung eines erfindungsgemäßen optischen Elements, insbesondere einer Kondensorlinse mit Freiformfläche,
- Fig. 4 -: ine schematische Darstellung des Auges eines Patienten in zwei verschiedenen Pupillenpositionen und
- Fig. 5 -: eine schematische Darstellung eines erfindungsgemäßen optischen Elements, insbesondere einer Retina-Schutzlinse mit Freiformfläche.

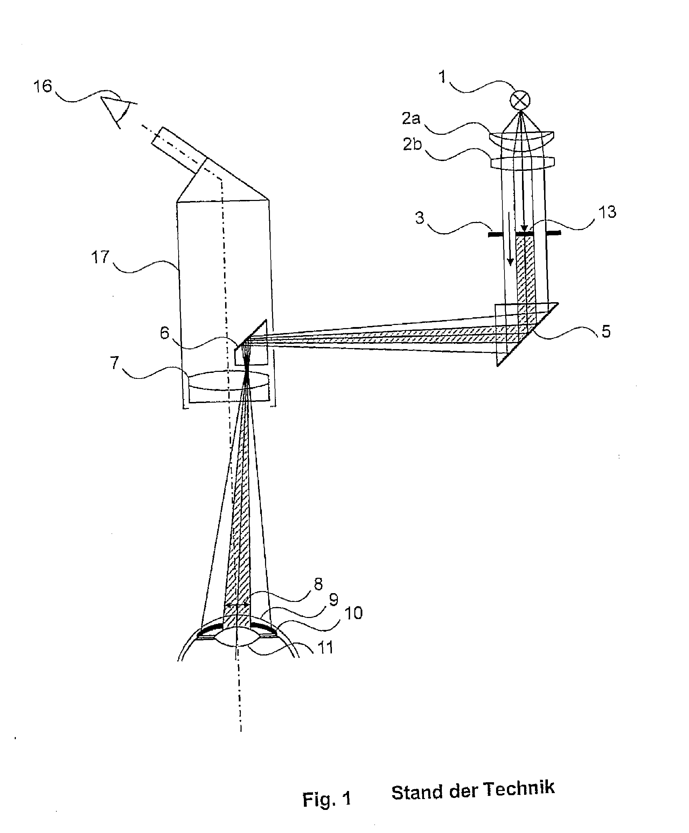

Fig. 1 zeigt eine Darstellung eines Beleuchtungsstrahlenganges eines Mikroskops 17 in Anwendung einer Retina-Schutzblende 13 nach dem Stand der Technik. Von einer Lichtquelle 1 wird Licht in den Beleuchtungsstrahlengang emittiert, der auch Kondensorlinsen 2a und 2b des Beleuchtungsstrahlenganges aufweist. In einer zur Objektebene konjugierten Ebene befindet sich eine Leuchtfeldblende 3 und in derselben Ebene eine Retina-Schutzblende 13, die das Licht im zentralen Bereich des Beleuchtungsstrahlenganges absorbiert. Über Umlenkelemente 5 und 6 und eine Optik 7 gelangt das verbleibende Licht auf das Operationsfeld. Die Abschattung 8, die durch die Retina-Schutzblende 13 erzeugt wird, überdeckt die Pupille in der Iris 10 eines Patientenauges. Somit trifft das Licht durch Teilbereiche der Hornhaut 9 hindurch eventuell auf die Iris 10, jedoch nicht auf die Pupille bzw. auf die Linse 11 des Patientenauges und somit auch nicht auf die Retina dieses Auges. Des Weiteren ist das Beobachterauge 16 eines Chirurgen dargestellt.

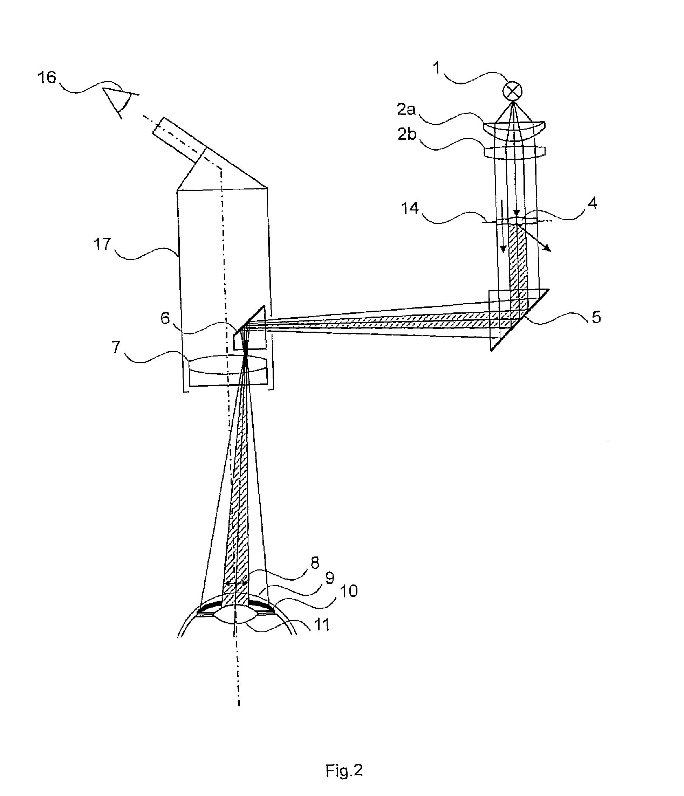

Fig. 2 ist eine Darstellung des Beleuchtungsstrahlenganges eines Mikroskops 17 in Anwendung eines erfindungsgemäßen optischen Elements 4, insbesondere einer speziellen Retina-Schutzlinse mit Freiformfläche. In der zur Objektebene 14 konjugierten Ebene befindet sich das optische Element 4, insbesondere die Retina-Schutzlinse, welche das Licht aus dem zentralen Bereich des Beleuchtungsstrahlenganges herauslenkt und nicht absorbiert. Das Licht verstärkt entweder die Lichtintensität im verbleibenden Bereich oder wird in für die Retina unproblematische andere seitliche Bereiche des Beleuchtungsstrahlenganges gelenkt. Die Abschattung 8, die durch die Freiformfläche der Retina-Schutzlinse erzeugt wird, bewirkt den Effekt, wie in der Beschreibung zu Fig. 1 erläutert.

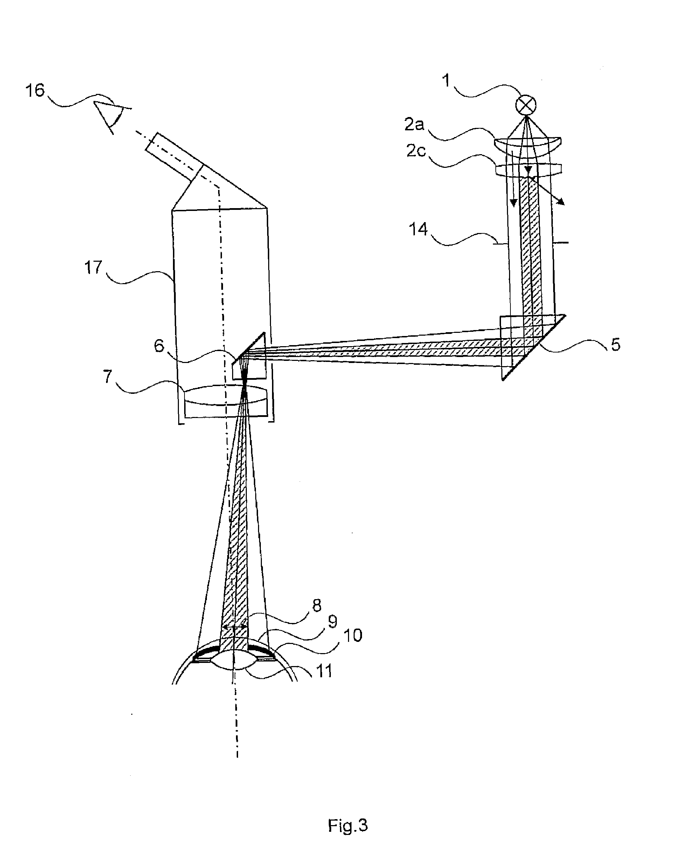

Fig. 3 ist eine Darstellung des Beleuchtungsstrahlenganges eines Mikroskops 17 in Anwendung eines erfindungsgemäßen optischen Elements, insbesondere einer Kondensorlinse 2c mit Freiformfläche. Außerhalb der zur Objektebene 14 konjugierten Ebene befindet sich eine Kondensorlinse 2c mit Freiformfläche, welche das Licht aus dem zentralen Bereich des Beleuchtungsstrahlenganges herauslenkt. Die Abschattung 8, die durch die Freiformfläche der Kondensorlinse 2c erzeugt wird, bewirkt den Effekt, wie in der Beschreibung zu Fig. 1 erläutert.

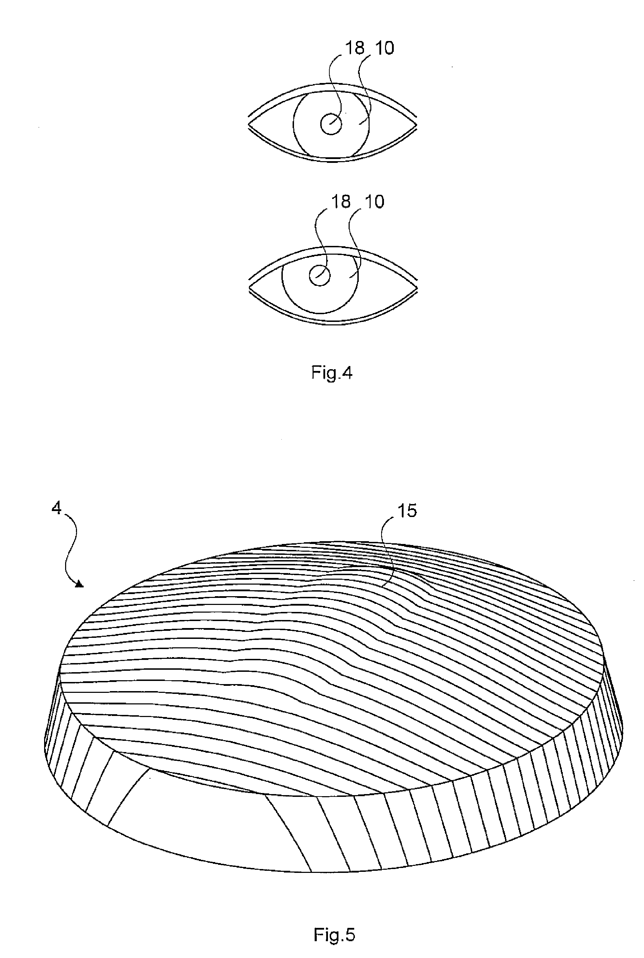

Fig. 4 zeigt eine Darstellung des Auges eines Patienten in zwei verschiedenen Pupillenpositionen. Im oberen Teil ist die Iris 10 mit der Pupille 18 zentral ausgerichtet und im unteren Teil nach links oben verrollt, also dezentral ausgerichtet.

Fig. 5 ist eine vergrößerte Darstellung eines erfindungsgemäßen optischen Elements 4, insbesondere einer Retina-Schutzlinse. Die beispielhaft dargestellte Freiformfläche 15 ist so ausgestaltet, dass sie das Licht aus dem zentralen Leuchtfeldbereich herauslenkt.

## Patentansprüche

1. Augen-, insbesondere Retina-Schutzvorrichtung für ein Operationsmikroskop mit einem Beleuchtungsstrahlengang, der ein Leuchtfeld für eine Objektebene (14) definiert, und einem optischen Element (4) im Beleuchtungsstrahlengang, **dadurch gekennzeichnet, dass** das optische Element (4) derart ausgebildet ist, dass es Licht aus dem zentralen oder einem dezentralen Bereich des Leuchtfeldes herauslenkt.

2. Augen-, insbesondere Retina-Schutzvorrichtung nach Anspruch 1, **dadurch gekennzeichnet, dass** das optische Element aus einer optischen Linse (4) besteht.

3. Augen-, insbesondere Retina-Schutzvorrichtung nach einem der vorhergehenden Ansprüche, **dadurch gekennzeichnet, dass** die brechenden Flächen der Linse (4) seitlich des zentralen oder dezentralen Bereichs einen unendlichen Radius aufweisen.

4. Augen-, insbesondere Retina-Schutzvorrichtung nach einem der vorhergehenden Ansprüche, **dadurch gekennzeichnet, dass** das optische Element (4) wenigstens eine Freiformfläche (15) aufweist.

5. Augen-, insbesondere Retina-Schutzvorrichtung nach einem der vorhergehenden Ansprüche, **dadurch gekennzeichnet, dass** die Linse (4) im zentralen oder in einem dezentralen Bereich des Beleuchtungsstrahlenganges in einer zur Objektebene (14) konjugierten Ebene angeordnet ist.

6. Augen-, insbesondere Retina-Schutzvorrichtung nach einem der vorhergehenden Ansprüche, **dadurch gekennzeichnet, dass** das optische Element wenigstens eine Kondensorlinse (2c) außerhalb einer zur Objektebene (14) konjugierten Ebene umfasst, an der wenigstens eine Freiformfläche (15) ausgestaltet ist.

7. Augen-, insbesondere Retina-Schutzvorrichtung nach einem der vorhergehenden Ansprüche, **dadurch gekennzeichnet, dass** das optische Element (4) aus dem Beleuchtungsstrahlengang - gegebenenfalls ferngesteuert - entfernbar ist.

8. Augen-, insbesondere Retina-Schutzvorrichtung nach einem der vorhergehenden Ansprüche, **dadurch gekennzeichnet, dass** das optische Element (4) aus dem Beleuchtungsstrahlengang elektrisch oder elektronisch angesteuert motorisch entfernbar ist.

9. Optisches Element (4) mit einer Freiformfläche für einen Beleuchtungsstrahlengang, der ein Leuchtfeld definiert, **dadurch gekennzeichnet, dass** das optische Element (4) so ausgebildet ist, dass es im Anwendungsfall Licht aus dem zentralen oder einem dezentralen Bereich eines Leuchtfeldes heraus in den verbleibenden Bereich desselben Leuchtfeldes hineinlenkt.

10. Optisches Element (4) nach Anspruch 9, **dadurch gekennzeichnet, dass** es im zentralen oder dezentralen Bereich des Beleuchtungsstrahlenganges eines Operationsmikroskops in einer zur Objektebene (14) konjugierten Ebene angeordnet ist.

11. Optisches Element (4) nach einem der Ansprüche 9 oder 10, **dadurch gekennzeichnet, dass** es aus dem Beleuchtungsstrahlengang - vorzugsweise motorisch gesteuert - entfernbar ist.

12. Verwendung eines optischen Elements mit Freiformfläche (15) als Augenschutzvorrichtung, vorzugsweise in einem optischen Vergrößerungsgerät.
